# EUROPEAN PATENT SPECIFICATION

(11) **EP 0 390 206 B1**
(45) Date of publication and mention of the grant of the patent: **18.09.1996**
(21) Application number: 90106181.2
(22) Date of filing: 30.03.1990
(51) Int. Cl.: C08J 3/09, A61K 7/00

(54) **Stable perfluoropolyether emulsions**
Beständige Perfluoropolyätheremulsionen
Emulsions de perfluoropolyéther stables

(30) Priority: 31.03.1989 IT 1997489
(43) Date of publication of application: 03.10.1990
(73) Proprietor: AUSIMONT S.p.A., I-20100 Milano (IT)
(72) Inventor: Brunetta, Fabio, Dr., I-31041 Cornuda, Treviso (IT); Pantini, Giovanni, Dr., I-20121 Milan (IT)
(74) Representative: Barz, Peter, Dr.

(56) References cited:
- EP-A- 0 196 904
- DE-A- 2 224 182
- US-A- 2 808 384
- DATABASE WPIL, abs.no.85-084003, Derwent Publications Ltd, LONDON, GB ; & JP-A-60 034 730
- DATABASE WPIL, abs.no.85-253804, Derwent PubliCations Ltd, London, GB ; & JP-A- 60 169 429

## Description

The present invention relates to stable emulsions of perfluoropolyethers.

In particular, the present invention relates to stable biphasic emulsions comprising a liquid perfluoropolyether component and an organic hydroxylated hydrating and/or wetting agent.

The emulsions forming the object of the present invention may be utilized in several known industrial fields for perfluoropolyethers, and in particular in the field of cosmetic and dermatological specialities and furthermore as so-called premixes in the preparation of the above specialities.

The use of perfluoropolyethers as ingredients of various cosmetic and dermatological preparations is known as such. Said perfluoropolyethers of fer the possibility of obtaining a protective film on the skin, which film does not interfere with the physiological functions of the skin (such as transpiration, etc.). Nevertheless, difficulties have been encountered in the production of corresponding cosmetic preparations which are due to the fact that perfluoropolyethers in general are insoluble in the raw materials utilized in the cosmetic industry, or, at least, it may happen that the performance of the corresponding product is impaired by a non-homogeneous distribution of the perfluoropolyethers in the cosmetic preparation. In fact, the perfluoropolyethers are completely insoluble in all organic substances, except in those having a high fluorine content, wherefore the general problem of obtaining dispersions of perfluoropolyethers in organic or aqueous-organic liquids (the perfluoropolyethers are liquids) arises.

It is known to prepare oil/water emulsions in which the oil consists of perfluorinated compounds. These emulsions have been prepared mainly for the preparation of synthetic plasma, taking advantage of the high solubility of oxygen and carbon dioxide in the perfluorinated compounds which thus act as oxygen carriers. The utilized perfluorinated compounds are perfluorinated cycloalkanes (preferably having two or more condensed cycles.), perfluorinated heterocyclic compounds and perfluorinated amines. The best emulsifiers have proven to be the non-ionic emulsifiers and particularly the so-called pluronic emulsifiers (non-ionic emulsifiers having a chemical structure of polyalkyloxanes and prepared by starting from mixtures of ethylene oxide and propylene oxide in a suitable ratio). Alternatively, also perfluorinated emulsifiers have been used in order to obtain emulsions and microemulsions. The applications of said emulsions are, however, limited due to the very presence of said surfactants.

The above technology has proven to be not very suitable for compounds having a perfluoropolyether structure, especially if the molecular weight of said compounds is higher than 1000. In the preparation of emulsions of the latter, two main difficulties have been encountered, i.e., the choice of an effective emulsifier and the obtainment of sufficiently stable products.

On the other hand it is known to prepare cosmetic three-phase emulsions in which the perfluoropolyether is dispersed in an oil-in-water or water-in-oil emulsion or in a crosslinking solid phase suspended in an organic liquid phase (see EP-A-196 904).

So far no biphasic systems have been described which consist of two liquids, one of which is a perfluoropolyether and the other is an organic, optionally aqueous, substance, in the form of a dispersion of one in the other, in order to form a stable emulsion, with the addition of common surfactants, particularly non-fluorinated surfactants.

JP-A-60-034730 (WPIL, Abstract 85-084003) discloses a perfluoropolyether emulsion to be added to water and oil repellent emulsions for use in the impregnation of cotton, wool and other fibers. Said emulsion also comprises certain perfluoroalkyl group containing compounds, non-ionic surfactants, hydrophilic organic solvents and water.

It is an object of the present invention to provide a biphasic emulsion in the form of a fine and homogeneous dispersion of perfluoropolyethers in an organic substance, optionally in the presence of water, which acts as a continuous phase, in the presence of conventional surfactants or emulsifiers.

Another object is the provision of the corresponding method of preparation, while further objects are the compositions containing the above stable emulsions and the use thereof in the cosmetic and dermatological field.

These and still other objects, which will become apparent from the following description, are achieved by means of stable emulsions of perfluoropolyether liquids having perfluoroalkyl end groups in a continuous phase selected from glycerol and solutions in certain hydrophilic solvents and/or water of certain polyalcohols and/or saccharides, as specified hereinafter, in the presence of conventional surfactants. In this way stable, also anhydrous, emulsions may be obtained, which are utilizable in the industrial fields which are known to make use of perfluoropolyethers. In particular, due to the film-forming characteristics of the perfluoropolyether component, the emulsions of the present invention may be used in compositions, creams, pastes, pseudo-solid emulsions, and the like, to which they impart the property of forming a transparent, water-repellent and lipo-repellent liquid film, also endowed with permeability to oxygen and other gases, which remains on the surface onto which it is applied for relatively long periods of time.

The characteristic water- and lipo-repellent properties of the liquid film render said compositions particularly suitable for applications in the field of protective preparations and for highly effective applications in cosmetology and/or dermatology. In comparison with conventional preparations, those having a water- and lipo-repellent action afford the important advantage of being long lasting while effective. In the skin-rehydration treatments it is highly desirable to have available stable, cosmetically acceptable (non-greasy, non-tacky, etc.) compositions which, besides maintaining in the subcutaneous layers water amounts which are sufficient for retaining elasticity, are also capable of exerting a "barrier" effect towards the outside without, however, adversely affecting the skin transpiration. The compositions of the invention, thanks to the perfluoropolyether component contained therein as a stable emulsion, allow to achieve these objects.

The stable biphasic emulsions according to the present invention show a fine and homogeneous, stable dispersion of at least one perfluoropolyether having perfluoroalkyl end groups in a continuous phase of a polyhydroxylated compound containing at least three hydroxy groups and selected from glycerol and solutions of polyalcohols containing from 3 to 12 carbon atoms and/or saccharides in certain hydrophilic solvents and/or water, in the presence of usual surfactants or emulsifiers. The perfluoropolyethers having perfluoroalkyl end groups, i.e. free from functional groups, are well-known compounds, which are described, along with the methods for preparing them, in several documents, among others, GB-A-1,104,482, US-A-3,242,218, 3,665,041, 3,715,378, 4,523,039, EP-A-148,482, 151,877 and 191,490 and WO 87/00538 and WO 87/02992.

Examples of suitable perfluoropolyethers are those which are characterized by the presence of one or more of the following units:
a) (CF₂-CF₂O)
b) (CF₂O)
c) (C₃F₆O), simplified formula for 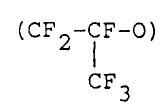
d) (CF₂O-CF₂-CF₂O)
e) (CF₂-CF₂-CF₂O)
f) 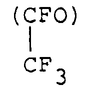
g) 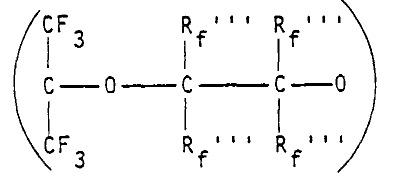
wherein the groups R_{f}"', the same or different from one another, are fluorine atoms or (preferably C₁₋₃-)perfluoroalkyl groups.

Preferably the perfluoropolyethers suitable for the present invention exhibit the following individual perfluorooxyalkylene units or the following combinations of perfluoro-oxyalkylene units:
I) (CF₂-CF₂O) and (CF₂O), said units being statistically distributed along the perfluoropolyether chain; or
II) 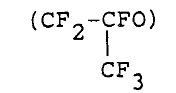 and (CFXO), wherein X is F or CF₃, said units being statistically distributed along the chain; or
III) (CF₂-CF₂O), 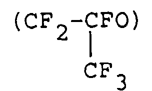 and (CFXO) in which X is F or CF₃, said units being statistically distributed along the chain; or
IV) 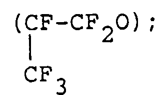 or
V) (CF₂-CF₂-CF₂O); or
VI) (CF₂-CF₂O); or
VII) 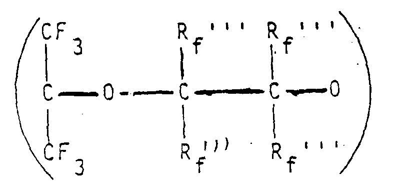 wherein the groups R_{f}''' are defined as above;
VIII) (CF₂O-CF₂-CF₂O).

Suitable perfluoropolyethers are also those which contain perfluorooxetane rings of formula 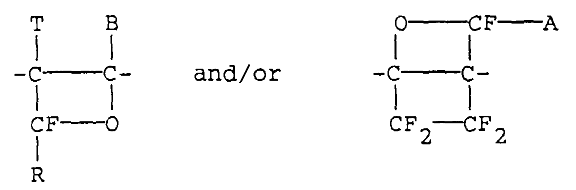 wherein T, B and R, the same or different from one another, are perfluorooxyalkyl, perfluoropolyoxyalkyl or perfluoroalkyl radicals and A is a perfluorooxyalkyl, perfluoropolyoxyalkyl or perfluoroalkyl radical.

Examples of suitable perfluoropolyethers having perfluorooxyalkylene units belong to the following classes:
A) 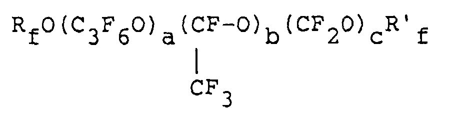 wherein: R_{f} and R'_{f}, the same or different from each other, are selected from CF₃, C₂F₅ and C₃F₇; the units C₃F₆O (oxytrifluoromethyltrifluorcethylene), 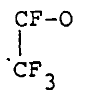 and CF₂-O are statistically distributed along the chain;
   a is an integer; and
   b and c are integers or zero;
   provided that when the sum (b+c) is different from zero, the ratio $\frac{\text{a}}{\text{b+c}}$ ranges from 5 to 40;
B) CF₃O(C₂F₄O)_{d}(CF₂O)ₑCF₃
   wherein the units C₂F₄O and CF₂O are statistically distributed along the chain; d and e are integers and the ratio d/e ranges from 0.3 to 5;
C) CF₃O(C₃F₆O)_{f}(C₂F₄O)_{g}(CFXO)ₕCF₃
   wherein the units C₃F₆O, C₂F₄O and CFXO are statistically distributed along the chain;
   X is F or CF₃;
   f, g and h are integers;
   the ratio $\frac{\text{f}}{\text{g+h}}$ varies from 1 to 50, and
   the ratio $\frac{\text{g}}{\text{h}}$ varies from 1 to 10;
D) R³_{f}O(CF₂CF₂CF₂O)ⱼR⁴_{f}
   wherein R³_{f} and R⁴_{f}, the same or different from each other, are CF₃ or C₂F₅ and j is an integer.

The perfluoropolyethers for use in the present invention usually have a number average molecular weight ranging from about 500 to about 20,000, particularly from about 1,000 to about 10,000.

As already mentioned above, the perfluoropolyethers used according to the present invention are dispersed, in the form of an emulsion, in a continuous phase of a polyhydroxylated compound containing at least three hydroxy groups and selected from glycerol as such and C₃₋₁₂-polyalcohols and saccharides , preferably in the absence of hydrogenated carbon atoms, in concentrated solution (50 to 80% by weight) in a hydrophilic solvent (specified below) and/or water.

Glycerol is the preferred compound and can be used just as it is commercially available, generally containing up to 5% of water.

Any other C₃₋₁₂-polyalcohol containing at least 3 hydroxy groups can be utilized, only provided that it is liquid under ambient conditions or that it is at least soluble in the hydrophilic solvent and/or water to form a 50 to 80% by weight solution.

The suitable polyalcohols are those which contain from 3 to 12 carbon atoms and at least 3 hydroxyl groups. Similarly, under the same condition of solubility in the hydrophilic solvent and/or water, it is possible to use (preferably C₄₋₁₈-) saccharides (from mono- to tri-saccharides) and/or mixtures thereof (e.g. mixtures obtainable from the hydrolytic degradation of polysaccharides such as cellulose and starch).

The hydrophilic solvent is selected from propylene glycol, glycerol itself, ethanol, n- and di-propanol, THF, dioxane, diglyme and mixtures thereof, optionally in aqueous form.

Of course, glycerol cannot be the polyhydroxylated compound and said hydrophilic solvent at the same time.

The solvent usually employed is water.

The hydrophilic solvent and/or water are preferably used in the lowest amount sufficient for obtaining a concentrated solution (syrup) of the polyalcohol or saccharide compound, but in any case in an amount such that a 50 to 80% by weight solution of the polyalcohol or saccharide compound is afforded.

The concentrated aqueous solutions (syrups) of polyalcohols and saccharides (sugars) available on the market may also be employed.

The concentration of the solutions ranges from 50 to 80% by weight. Glycerol may be used as such because it already is in the liquid state.

The following polyalcohols and saccharides have proven to be particularly effective: glycerol, xylitol, mannitol, sorbitol, glucose, fructose, saccharose, maltitol, dimeric compounds of glycerol (di-glycerol or bis(2,3-di-hydroxy propyl) ether), solid water-soluble polyhydroxylated compounds such as sugars and glycerol condensation products (e.g. triglycerol and tetraglycerol).

These are known compounds which are in the form of crystalline powders soluble both in water and in hydroxylated solvents.

The formation of the emulsions may be carried out in the presence of conventional surfactants, preferably of the cosmetic type. The surfactants of the cationic, anionic, amphoteric and non-ionic type have proven to be useful compounds, the preferred ones being those of the ionic type.

Among the others, the following ones also have proven to be effective:
- sodium lauryl sulphate (solution at 28%), commercially available as Texapon ® N40 (Henkel),
- sulphosuccinate (sulphosuccinic hemiester) (solution at 30%), commercially available as Texapon ® SB 3 (Henkel),
- coco-amphocarboxyglycinate (solution at 40%), commercially available as Dehyton ® D (Henkel),
- potassium cetyl phosphate (solid product), commercially available as Amphisol ® K (Givaudan),
- sodium alkyl-polyoxyethylene-ether carboxylate, commercially available as Nikkol ® ECTD-3NEX (Nikko Chemicals),
- potassium benzalconium chloride (solution at 50%) (purchased from Res Pharma),
- alkyl amidopropyl betaine (solution at 40%), commercially available as Dehyton ® K (Henkel).

As already mentioned, sufficiently stable emulsions have been prepared also with non-ionic emulsifiers such as:
- cetyl-stearyl ethoxylated alcohol, e.g. Emulgin ® B1 (Henkel),
- sorbitan-ethoxylate(20)-mono-oleate, e.g. Tween ® 20 (ICI Speciality Chemicals).

The ratios of the components of the stable emulsion according to the present invention can be defined as follows:
1. In the case of using only glycerol as the continuous phase:
   a) perfluoropolyether: from 0.01 to 75%, preferably from 0.1 to 75% by weight, based on the total weight of the emulsion;
   b) surfactant: from 0.01 to 30%, preferably from 0.01 to 5% by weight, based on the total weight of the emulsion and most preferably the lowest amount possible;
   c) glycerol: the balance to 100% by weight.
2. In the case of using polyalcohols or saccharides (in the solid state), dissolved in the above hydrophilic solvent and/or H₂O :
   a) perfluoropolyether: from 0.01 to 80%, preferably from 0.01 to 50% by weight, based on the total weight of the emulsion;
   b) surfactant: from 0.01 to 30%, preferably from 0.01 to 5% by weight, based on the total weight of the emulsion;
   c) polyhydroxylated compound (polyalcohol and/or saccharide) expressed at 100% in in the form of a 50 to 80% by weight solution in the above hydrophilic solvent and/or water: the balance to 100%.

Within the limits of the above preferred ranges it is possible to obtain emulsions of the Newtonian type with particle sizes of the order of 0.5 to 0.8 µm.

The emulsions of the present invention may be prepared by adding the perfluoropolyether to a solution of a preferably ionic surfactant or emulsifier, as defined hereinbefore, in glycerol or in the polyalcohol and/or saccharide present as 50 to 80% by weight solution (syrup in H₂O) in the above ratios, maintaining the system under stirring, for example by means of a turboemulsifier Silverson L/2R at room temperature.

The emulsions of the present invention exhibit remarkable film-forming effects: the obtained liquid film is transparent and permeable to gases. Significant evidence of the waterproof effect is provided by applying a cream according to the invention onto the hands and then washing the hands. After washing, water glides away, leaving the skin dry.

Thanks to the above properties, the emulsions of the present invention are particularly suitable for applications in the field of cosmetology and dermatology.

Examples of these applications are:
a) as barrier creams and other protecting preparations (hand creams, ointments or pastes to prevent contact irritations and dermatitis; creams against dermatitis caused by household or work surfactants);
b) in paedo-cosmetology as protective creams or pastes for children;
c) in sun-protection products to prolong the action thereof;
d) as anti-wrinkle products and for decorative cosmetology, for example in products such as make-up foundation products, eyeshadows and the like. In this case, the presence of the fluorinated compound promotes the flowability and therefore facilitates the spreadability of the products, thereby preventing or minimizing unaesthetic caking of the product on the skin; in lipsticks and lip-glosses, for example, an improvement of both flowability and gloss is obtained;
e) as creams for massages; since the fluorinated compound is not absorbed by the skin, it also permits prolonged massages, thereby allowing the penetration of "active matter", if any;
f) in dermatological applications, as vehicle for medicaments.

The concentration of perfluoropolyether in cosmetic emulsions varies as a function of the type of end use, the number of daily applications and the application time. Said amount generally ranges from 0.2 to 0.5% for the anti-wrinkle creams to be utilized every day, up to 3 to 5% for highly protective creams. The persistence of the perfluoropolyether on the skin is rather long: removal occurs either by washing or by diffusion onto the clothes of by natural desquamation of the skin.

The following examples are given for illustrative purposes and are not to be considered as a limitation of the scope of the invention. Unless otherwise specified, all parts are parts by weight.

Furthermore, the parts of polyalcohols or of saccharides and of surfactant are indicated as such according to the indicated concentration.

The following perfluoropolyethers were used:
- Fomblin ® Hc/25 (Montefluos S.p.A.) (number average molecular weight) M.W. = 3200; kinematic viscosity = 250 mm²/s (cSt) (20°C);
- Fomblin ® HC/R (Montefluos S.p.A. M.W. = 6600; kinematic viscosity = 1500 mm²/s (cSt) (20°C);
- Galden ® D 03 (Montefluos S.p.A.) M.W. = 870; kinematic viscosity = 2.4 mm²/s (cSt) (25°C);
- Galden ® D 10 (Montefluos S.p.A.) M.W. = 1320; kinematic viscosity = 9 mm²/s (cSt) (25°C); all of the above products have the structure: 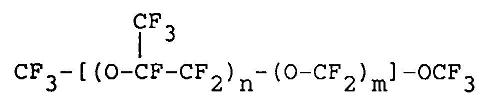 wherein n/m = 20-40.
- Fomblin ® Z/25 (Montefluos S.p.A.) M.W. = 9400; kinematic viscosity = 255 mm²/s (cSt) (20°C), having the structure:

   CF₃-[(O-CF₂-CF₂)ₚ-(O-CF₂)_{q}]-OCF₃

   wherein p/q = 0.6-0.7.
- Fomblin ® M30 (Montefluos S.p.A.) M.W. = 9400; kinematic viscosity = 310 mm²/s (cSt) (20°C), having the same structure as Fomblin ® Z/25 with p/q = 1.2
- Krytox ® 1525 (Du Pont) M.W. = 4600; kinematic viscosity = 261 mm²/s (cSt) (20°C), having the structure: 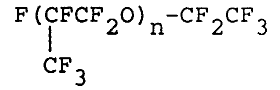 wherein n = 25-30.
- Demnum ® S-100 (Daikin) M.W. = 5600; kinematic viscosity = 250 mm²/s (cSt) (20°C), having the structure:

   F(CF₂CF₂CF₂O)ₙCF₂CF₃

   wherein n = 30-35.

The stability was measured on the individual emulsions prepared in a centrifuge operating at 4000 r.p.m., for 1 hour, and then subjected to an ageing test at room temperature for 3 months on a shelf, then in an oven at 100°C for 1 week and at 180°C for 3 hours.

### EXAMPLE 1

An emulsion was prepared by stirring, in a Silverson L/2R turboemulsifier at 5000/6000 r.p,m., 20 parts of perfluoropolyether Fomblin ® HC/25 in 80 parts of glycerol, in the presence of 2 parts of emulsifying surfactant Texapon ® N40 (solution at 28%). The following procedure was employed: the emulsifier was dissolved in glycerol and then the perfluoropolyether was added while stirring at room temperature for a few moments. An emulsion was obtained, which, subjected to the above stability tests, proved to be stable.

### EXAMPLES 2-5

Example 1 was repeated under the same conditions and with the same ingredients, but varying the amounts in the formulation as indicated in the following Table 1 along with the results.

**TABLE 1**

| Example | Glycerol (parts) | Fomblin® HC/25 (parts) | Stability |
|---|---|---|---|
| 2 | 90 | 10 | yes |
| 3 | 50 | 50 | yes |
| 4 | 30 | 70 | yes |
| 5 | 25 | 75 | yes |

### EXAMPLES 6-11

Examples 1 to 6 were repeated, but substituting Fomblin ® HC/R for Fomblin ® HC/25, thereby obtaining the results reported in the following Table 2.

**TABLE 2**

| Example | Glycerol (parts) | Fomblin® HC/R (parts) | Stability |
|---|---|---|---|
| 6 | 90 | 10 | yes |
| 7 | 80 | 20 | yes |
| 8 | 70 | 30 | yes |
| 9 | 50 | 50 | yes |
| 10 | 30 | 70 | yes |
| 11 | 25 | 75 | yes |

### EXAMPLES 12-16

Example 2 was repeated, varying the amount of emulsifier as indicated in Table 3.

**TABLE 3**

| Example | Glycerol (parts) | Fomblin® HC/25 (parts) | Texapon® N40 (parts) |
|---|---|---|---|
| 12 | 90 | 10 | 0.5 |
| 13 | 90 | 10 | 0.25 |
| 14 | 90 | 10 | 0.10 |
| 15 | 90 | 10 | 0.05 |
| 16 | 90 | 10 | 0.025 |

Stable emulsions were obtained.

### EXAMPLES 17-20

Example 1 was repeated, but glycerol was substituted by the following polyhydroxylated compounds (polyols or saccharides) in aqueous solution, according to the formulations indicated in Table 4.

**TABLE 4**

| Example | Polyhydroxylated compound (parts) | | Fomblin® HC/25 (parts) | Texapon® N40 (parts) |
|---|---|---|---|---|
| 17 | Maltitol | 90 | 10 | 0.025 |
| | (solution at 74%) | | | |
| 18 | Sorbitol | 90 | 10 | 2 |
| | (solution at 70%) | | | |
| 19 | Glucose | 90 | 10 | 2 |
| | (solution at 70%) | | | |
| 20 | Saccharose | 90 | 10 | 2 |
| | (solution at 70%) | | | |

The emulsions obtained were stable.

Analogous results were obtained when using xylitol and mannitol syrups.

### EXAMPLES 21-23

Example 1 was repeated, substituting other ionic emulsifiers for Texapon® N40, according to the formulations indicated in Table 5.

**TABLE 5**

| Example | Glycerol (parts) | Fomblin® HC/25 (parts) | Emulsifier (parts) |
|---|---|---|---|
| 21 | 90 | 10 | Amphisol® K (solid) 0.25 |
| 22 | 80 | 20 | Nikkol® ECTD-3NEX (semisolid) 1 |
| 23 | 90 | 10 | Benzalconium 25 chloride K (solution at 50%) |

Stable emulsions were obtained.

Analogous results were obtained when using the following ionic surfactants: Dehyton ® D; Texapon ® SB3 and Dehyton ® K.

### EXAMPLE 24

Example 1 was repeated, using the following formulation:
- Maltitol (solution at 74%) 40 parts
- Fomblin ® HC/25 20 parts
- Dehyton ® K (solution at 40%) 2 parts

A stable emulsion was obtained.

### EXAMPLES 25-26

Example 1 was repeated, using the following formulations comprising a non-ionic emulsifier, according to Table 6.

**TABLE 6**

| Example | Glycerol (parts) | Fomblin® HC/25 (parts) | Emulsifier (parts) | |
|---|---|---|---|---|
| 25 | 90 | 10 | Tween® 20 | 1 |
| 26 | 90 | 10 | Emulgin® B1 | 2 |

Shelf-stable emulsions were obtained.

### EXAMPLES 27-32

Example 1 was repeated, but substituting other perfluoropolyethers for Fomblin ® HC/25, according to the formulations indicated in Table 7.

**TABLE 7**

| Example | Glycerol (parts) | Texapon® N40 (parts) | Perfluoropolyether (parts) | |
|---|---|---|---|---|
| 27 | 90 | 2 | Demnum® S/100 | 2 |
| 28 | 90 | 2 | Krytox® 1525 | 10 |
| 29 | 90 | 1 | Galden® D03 | 10 |
| 30 | 74 | 1 | Galden® D10 | 25 |
| 31 | 90 | 2 | Fomblin® Z/25 | 10 |
| 32 | 90 | 2 | Fomblin® 30M | 10 |

Stable emulsions were obtained.

### EXAMPLES 33-36

Example 1 was repeated with the formulations indicated in Table 8.

**TABLE 8**

| Example | Glycerol (parts) | Fomblin® HC/25 (parts) | Texapon® N40 (parts) |
|---|---|---|---|
| 33 | 38 | 60 | 2 |
| 34 | 36 | 62 | 2 |
| 35 | 34 | 64 | 2 |
| 36 | 32 | 64 | 4 |

Stable emulsions were obtained.

### EXAMPLE 37

Example 1 was repeated with the following formulation:
- Maltitol 58 parts
- Fomblin ® HC/25 40 parts
- Texapon ® N40 2 parts (solution at 28%).

A stable emulsion was obtained.

### EXAMPLE 38

Example 1 was repeated with the following formulation:
- di-glycerol 70 parts
- Fomblin ® HC/R 28 parts
- Texapon ® N40 2 parts

A stable emulsion was obtained.

### EXAMPLE 39

Example 1 was repeated, using the following formulation:
- glycerol 50 parts
- propylene glycol 28 parts
- Fomblin ® HC/25 20 parts
- Texapon ® N40 2 parts

A stable emulsion was obtained.

### EXAMPLE 40

### Preparation of creams according to the perfluoropolyether pre-emulsification technique

| Hydrating cream | | (% by weight) |
|---|---|---|
| a) | PEG-8 C₁₂₋₁₈ alkyl ester | 6.0 |
| | PEG-20 methyl-glucose sesquistearate | 1.2 |
| | Isopropyl stearate | 5.0 |
| | Cetyl alcohol | 3.0 |
| | Stearic acid | 1.0 |
| | Octyl stearate | 6.0 |
| | Almond oil | 2.0 |
| | Antioxidants | as sufficient |
| b) | Water, balance to | 100.0 |
| | Natural hydrating factors | 1.0 |
| c) | Emulsion (example 1) | 4.0 |
| d) | Perfume, preserving agents and sequestering agents | as sufficient |

### Procedure

The emulsion of Fomblin ® HC/25 in glycerol, as specified in example 1(c), was utilized.

a) and b) were separately heated to 75°C. b) was added to a) under stirring.

Emulsion c) was added at room temeprature, stirring was continued and d) was added.

A centrifugation-stable cream was obtained, in which Fomblin ® HC/25 was present in a finely dispersed form (below 1 µm).

### EXAMPLE 41

| Sun emulsion | | (% by weight) |
|---|---|---|
| a) | Stearic acid | 4.0 |
| | Cetyl alcohol | 1.0 |
| | Caprylic acid/capric acid esterified with coco alcohol | 6.0 |
| | Tocopherol acetate | 2.5 |
| | Dimeticone | 0.3 |
| | Octyl methoxycinnamate | 6.0 |
| | Butylmethoxybenzoyl methane | 1.5 |
| b) | Potassium cetyl phosphate (Amphisol ® K) | 2.0 |
| c) | Potassium hydroxide | 0.15 |
| | Pantenol (aminoalcohol) | 2.0 |
| d) | Gelling agent (acrylic polymer: Carbomer ® 940) | as sufficient |
| | Water, balance to | 100.0 |
| e) | Emulsion (example 1) | 7.0 |
| f) | Perfume, sequestering agents and preserving agents | as sufficient |

### Procedure

a) was heated to 85°C in a (planetary type) mixer, then b) was added. After a homogeneous solution was obtained, c), preheated to 75°C, was added and mixing was continued. The whole mixture was allowed to cool to 40°C. d) was added.

The emulsion of Fomblin ® HC/25 in glycerol, as specified in example 1 (e), was utilized.

e) was added under stirring until reaching room temperature.

f) was then added.

### EXAMPLE 42

| Barrier cream | | (% by weight) |
|---|---|---|
| a) | PEG-8 C₁₂₋₁₈ alkyl ester | 10.0 |
| | Glyceryl stearate and PEG-100 stearate | 3.0 |
| | Octyl stearate | 10.0 |
| | Cetyl alcohol | 3.0 |
| b) | Water, balance to | 100.0 |
| c) | Emulsion (example 9) | 6.0 |
| d) | Perfume, preserving agents and sequestering agents | as sufficient |

### Procedure

The emulsion of Fomblin ® HC/R in glycerol, as specified in example 9 (c), was utilized.

a) and b) were heated separately to 75°C. b) was added to a) under stirring.

At room temperature (or indifferently also at elevated temperature) emulsion c) was added. Stirring was continued and d) was added.

### EXAMPLE 43

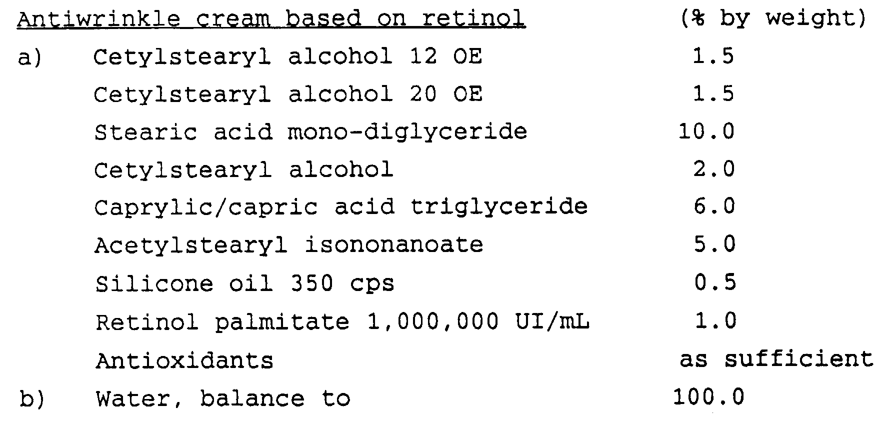

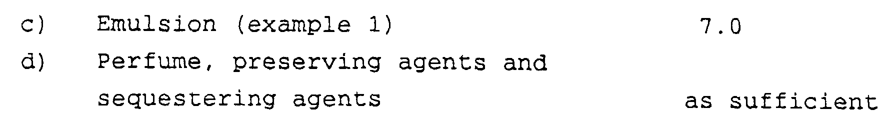

### Procedure

The aqueous phase and the oily phase were heated separately to 75°C, emulsifying them by means of a turbine and cooling them under stirring. The thermolabile components, the Fomblin ® emulsion and the perfume were added at 40°C, and mixing was continued until reaching room temperature.

## Claims

1. Biphasic emulsion of perfluoropolyethers having perfluoroalkyl end groups, consisting of:
(a) from 0.1 to 75% by weight of perfluoropolyether;
(b) from 0.01 to 30% by weight of surfactant and
(c) up to 99.89% by weight, with respect to the total weight of the emulsion, of glycerol.

2. Biphasic emulsion of perfluoropolyethers having perfluoroalkyl end groups, consisting of:
(a) from 0.01 to 80% by weight of perfluoropolyether;
(b) from 0.01 to 30% by weight of surfactant and
(c) up to 99.98% by weight, with respect to the total weight of the emulsion, of a solution containing 50 to 80% by weight of at least one polyhydroxylated compound containing at least three hydroxy groups and selected from polyalcohols containing from 3 to 12 carbon atoms and saccharides in a solvent selected from ethanol, n- and iso-propanol, propylene glycol, glycerol, tetrahydrofuran, dioxane, diglyme and mixtures thereof and/or in water, provided that the polyhydroxylated compound and said solvent are not glycerol at the same time.

3. Emulsion according to claim 1, consisting of:
from 0.1 to 75% by weight of component (a);
from 0.01 to 5% by weight of component (b); and
up to 99.89% by weight of component (c).

4. Emulsion according to claim 2, consisting of:
from 0.01 to 50% by weight of component (a),
from 0.01 to 5% by weight of component (b); and
up to 99.98% by weight of component (c).

5. Emulsion according to any one of the preceding claims, wherein the perfluoropolyether is selected from one or more perfluoropolyethers containing one or more of the following units:
a) (CF₂-CF₂O)
b) (CF₂O)
c) 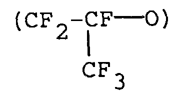
d) (CF₂O-CF₂-CF₂O)
e) (CF₂-CF₂-CF₂O)
f) 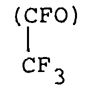
g) 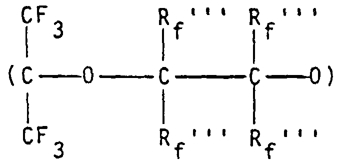
wherein the groups R_{f}''', the same or different from each other, are fluorine atoms or perfluoroalkyl groups.

6. Emulsion according to any one of the preceding claims, wherein the perfluoropolyether is selected from those which contain the following perfluorooxyalkylene units, either alone or combined with one another:
I) (CF₂-CF₂O) and (CF₂O), said units being statistically distributed along the perfluoropolyether chain;
II) 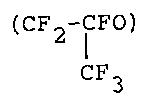 and (CFXO), wherein X is F or CF₃, said units being statistically distributed along the perfluoropolyether chain;
III) (CF₂-CF₂O), 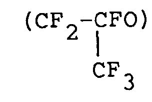 and (CFXO), wherein X is F or CF₃, said units being statistically distributed along the perfluoropolyether chain;
IV) 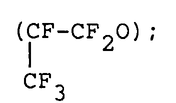
V) (CF₂-CF₂-CF₂O);
VI) (CF₂-CF₂O);
VII) 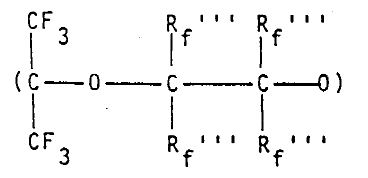 wherein the groups R_{f}''', the same or different from one another, are fluorine atoms or perfluoroalkyl groups;
VIII) (CF₂O-CF₂-CF₂O).

7. Emulsion according to any one of the preceding claims, wherein the perfluoropolyether is selected from those containing perfluorooxetane rings of formula: 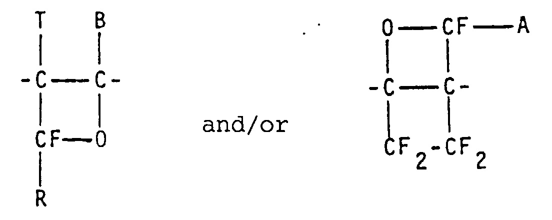 wherein T, B and R, the same or different from one another, and A are perfluorooxyalkyl, perfluoropolyoxyalkyl or perfluoroalkyl radicals.

8. Emulsion according to any one of claims 1 to 6, wherein the perfluoropolyether is selected from those belonging to the following classes:
A) 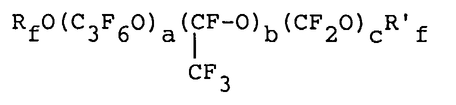 wherein R_{f} and R'_{f}, the same or different from each other, are selected from CF₃, C₂F₅ and C₃F₇;
the units in brackets are statistically distributed along the perfluoropolyether chain;
a is an integer and b and c are integers or zero; provided that when the sum (b+c) is different from zero, the ratio $\frac{\text{a}}{\text{b+c}}$ ranges from 5 to 40;
B) CF₃O(C₂F₄O)_{d}(CF₂O)ₑCF₃
wherein the units in brackets are statistically distributed along the chain; d and e are integers and the ratio d/e ranges from 0.3 to 5;
C) CF₃O(C₃F₆O)_{f}(C₂F₄O)_{g}(CFXO)ₕCF₃
wherein the units in brackets are statistically distributed along the chain;
X is F or CF₃;
f, g and h are integers;
the ratio $\frac{\text{f}}{\text{g+h}}$ ranges from 1 to 50,
and the ratio $\frac{\text{g}}{\text{h}}$ ranges from 1 to 10;
D) R³_{f}O(CF₂CF₂CF₂O)ⱼR⁴_{f}
wherein R³_{f} and R⁴_{f}, the same or different from each other, are CF₃ or C₂F₅ and j is an integer.

9. Emulsion according to any one of the preceding claims, wherein the perfluoropolyether has a number average molecular weight of from 500 to 20,000, particularly from 1,000 to 10,000.

10. Emulsion according to any one of claims 2 and 4 to 9, wherein said polyhydroxylated compound is selected from xylitol, mannitol, sorbitol, glucose, maltitol, saccharose, fructose, diglycerol, triglycerol and tetraglycerol.

11. Emulsion according to any one of the preceding claims, wherein the surfactant is selected from cationic, anionic, amphoteric and non-ionic surfactants.

12. Compositions for cosmetology and dermatology, containing an emulsion according to any one of the preceding claims.

## Patentansprüche

1. Zweiphasige Emulsion von Perfluorpolyethern mit Perfluoralkyl-Endgruppen, bestehend aus:
(a) 0,1 bis 75 Gewichts-% Perfluorpolyether;
(b) 0,01 bis 30 Gewichts-% Tensid und
(c) bis zu 99,89 Gewichts-%, bezogen auf das Gesamtgewicht der Emulsion, Glycerin.

2. Zweiphasige Emulsion von Perfluorpolyethern mit Perfluoralkyl-Endgruppen, bestehend aus:
(a) 0,01 bis 80 Gewichts-% Perfluorpolyether;
(b) 0,01 bis 30 Gewichts-% Tensid und
(c) bis zu 99,98 Gewichts-%, bezogen auf das Gesamtgewicht der Emulsion, einer Lösung, die 50 bis 80 Gewichts-% mindestens einer polyhydroxylierten Verbindung, die mindestens drei Hydroxygruppen enthält und aus Polyalkoholen, die 3 bis 12 Kohlenstoffatome enthalten, und Sacchariden ausgewählt ist, in einem aus Ethanol, n- und Isopropanol, Propylenglycol, Glycerin, Tetrahydrofuran, Dioxan, Diglyme und Mischungen davon und/oder in Wasser enthält, mit der Maßgabe, daß die polyhydroxylierte Verbindung und das Lösungsmittel nicht gleichzeitig Glycerin sind.

3. Emulsion nach Anspruch 1, bestehend aus:
0,1 bis 75 Gewichts-% Komponente (a);
0,01 bis 5 Gewichts-% Komponente (b); und
bis zu 99,89 Gewichts-% Komponente (c).

4. Emulsion nach Anspruch 2, bestehend aus:
0,01 bis 50 Gewichts-% Komponente (a);
0,01 bis 5 Gewichts-% Komponente (b); und
bis zu 99,98 Gewichts-% Komponente (c).

5. Emulsion nach irgendeinem der vorangehenden Ansprüche, in welcher der Perfluorpolyether aus einem oder mehreren Perfluorpolyethern ausgewählt ist, die eine oder mehrere der folgenden Einheiten enthalten:
a) (CF_{**2**}-CF_{**2**}O)
b) (CF_{**2**}O)
c) 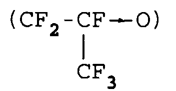
d) (CF_{**2**}O-CF_{**2**}-CF_{**2**}O)
e) (CF_{**2**}-CF_{**2**}-CF_{**2**}O)
f) 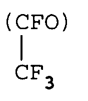
g) 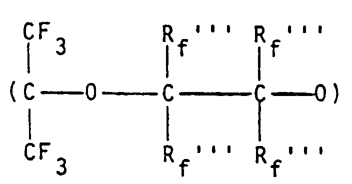
worin die Gruppen R_{f}''', gleich oder verschieden voneinander, für Fluoratome oder Perfluoralkylgruppen stehen.

6. Emulsion nach irgendeinem der vorangehenden Ansprüche, in welcher der Perfluorpolyether aus denjenigen ausgewählt ist, die die folgenden Perfluoroxyalkylen-Einheiten entweder allein oder in Kombination miteinander enthalten:
I) (CF_{**2**}-CF_{**2**}O) und (CF_{**2**}O), wobei diese Einheiten statistisch entlang der Perfluorpolyetherkette verteilt sind;
II) 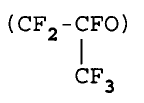 und (CFXO), worin X für F oder CF₃ steht, wobei die Einheiten statistisch entlang der Perfluorpolyetherkette verteilt sind;
III) (CF₂-CF₂O), 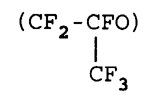 und (CFXO), worin X für F oder CF₃ steht, wobei diese Einheiten statistisch entlang der Perfluorpolyetherkette verteilt sind;
IV) 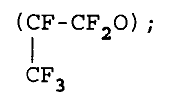
V) (CF_{**2**}-CF_{**2**}-CF_{**2**}O);
VI) (CF_{**2**}-CF_{**2**}O);
VII) 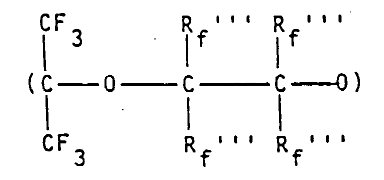 worin die Gruppen R_{f}''', gleich oder verschieden voneinander, für Fluoratome oder Perfluoralkylgruppen stehen;
VIII) (CF_{**2**}O-CF_{**2**}-CF_{**2**}O).

7. Emulsion nach irgendeinem der vorangehenden Ansprüche, in welcher der Perfluorpolyether aus denjenigen ausgewählt ist, die Perfluoroxetanringe der Formel: 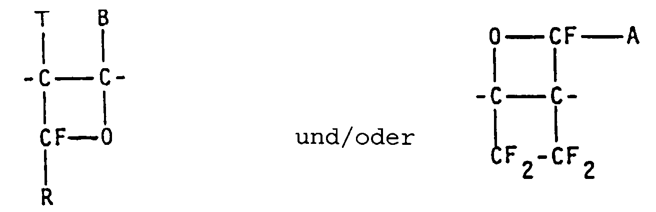 enthalten, worin T, B und R, gleich oder verschieden voneinander, und A für Perfluoroxyalkyl-, Perfluorpolyoxyalkyl- oder Perfluoralkyl-Reste stehen.

8. Emulsion nach irgendeinem der Ansprüche 1 bis 6, in welcher der Perfluorpolyether aus denjenigen ausgewählt ist, die zu den folgenden Klassen gehören: 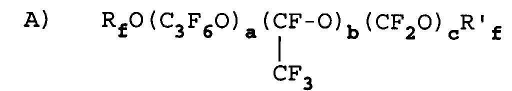
worin R_{f} und R'_{f}, gleich oder verschieden voneinander, aus CF_{**3**}, C_{**2**}F_{**5**} und C_{**3**}F_{**7**} ausgewählt sind;
die Einheiten in Klammern statistisch entlang der Perfluorpolyetherkette verteilt sind;
a eine ganze Zahl ist und b und c ganze Zahlen oder 0 bedeuten;
mit der Maßgabe, daß wenn die Summe (b + c) von null verschieden ist, das Verhältnis a/(b + c) im Bereich von 5 bis 40 liegt;
B) CF_{**3**}O(C_{**2**}F_{**4**}O)_{**d**}(CF_{**2**}O)_{**e**}CF_{**3**}
worin die Einheiten in Klammern statistisch entlang der Kette verteilt sind; d und e ganze Zahlen sind und das Verhältnis d/e im Bereich von 0,3 bis 5 liegt;
C) CF_{**3**}O(C_{**3**}F_{**6**}O)_{**f**}(C_{**2**}F_{**4**}O)_{**g**}) (CFXO)_{**h**}CF_{**3**}
worin die Einheiten in Klammern statistisch entlang der Kette verteilt sind;
X für F oder CF_{**3**} steht;
f, g und h ganze Zahlen sind;
das Verhältnis f/(g + h) im Bereich von 1 bis 50 liegt
und das Verhältnis g/h im Bereich von 1 bis 10 liegt;
D) R^{**3**}_{**f**}O(CF_{**2**}CF_{**2**}CF_{**2**}O)_{**j**}R^{**4**}_{**f**}
worin R^{**3**}_{**f**} und R^{**4**}_{**f**}, gleich oder verschieden voneinander, für CF_{**3**} oder C_{**2**}F_{**5**} stehen und j eine ganze Zahl ist.

9. Emulsion nach irgendeinem der vorangehenden Ansprüche, in welcher der Perfluorpolyether ein Zahlenmittel des Molekulargewichts von 500 bis 20000, insbesondere von 1000 bis 10000, aufweist.

10. Emulsion nach irgendeinem der Ansprüche 2 und 4 bis 9, in welcher die polyhydroxylierte Verbindung aus Xylit, Mannit, Sorbit, Glucose, Maltit, Saccharose, Fructose, Diglycerin, Triglycerin und Tetraglycerin ausgewählt ist.

11. Emulsion nach irgendeinem der vorangehenden Ansprüche, in welcher das Tensid aus kationischen, anionischen, amphoteren und nicht-ionischen Tensiden ausgewählt ist.

12. Zusammensetzungen für die Kosmetologie und Dermatologie, enthaltend eine Emulsion nach irgendeinem der vorangehenden Ansprüche.

## Revendications

1. Emulsion biphasique de perfluoropolyéthers ayant des groupes terminaux perfluoroalkyle, consistant en :
(a) de 0,1 à 75% en poids de perfluoropolyéther ;
(b) de 0,01 à 30% en poids d'agent tensio-actif ; et
(c) jusqu'à 99,89% en poids, par rapport au poids total de l'émulsion, de glycérol.

2. Emulsion biphasique de perfluoropolyéthers, ayant des groupes terminaux perfluoroalkyle, consistant en :
(a) de 0,01 à 80% en poids de perfluoropolyéther ;
(b) de 0,01 à 30% en poids d'agent tensio-actif ; et
(c) jusqu'à 99,98% en poids, par rapport au poids total de l'émulsion, d'une solution contenant 50 à 80% en poids d'au moins un composé polyhydroxylé contenant au moins trois groupes hydroxy et choisi parmi les polyalcools contenant de 3 à 12 atomes de carbone et les saccharides dans un solvant choisi parmi l'éthanol, le n- et l'iso-propanol, le propylène glycol, le glycérol, le tétrahydrofuranne, le dioxanne, la diglyme et les mélanges de ceux-ci et/ou dans l'eau, à la condition que le composé polyhydroxylé et ledit solvant ne soient pas en même temps le glycérol.

3. Emulsion selon la revendication 1, consistant en :
- de 0,1 à 75% en poids de composant (a) ;
- de 0,01 à 5% en poids de composant (b) ; et
- jusqu'à 99,89% en poids de composant (c).

4. Emulsion selon la revendication 2, consistant en :
- de 0,01 à 50% en poids de composant (a) ;
- de 0,01 à 5% en poids de composant (b) ; et
- jusqu'à 99,98% en poids de composant (c).

5. Emulsion selon l'une quelconque des revendications précédentes, dans laquelle le perfluoropolyéther est choisi parmi un ou plusieurs perfluoropolyéthers contenant une ou plusieurs des unités suivantes :
(a) (CF₂-CF₂O)
(b) (CF₂O)
(c) 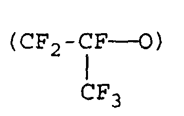
(d) (CF₂O-CF₂-CF₂O)
(e) (CF₂-CF₂-CF₂O)
(f) 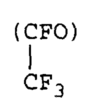
(g) 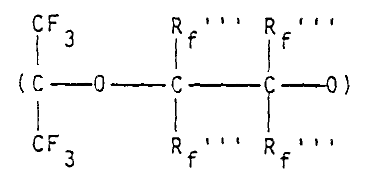
où les groupes R_{f}''', identiques ou différents les uns des autres, représentent des atomes de fluor ou des groupes perfluoroalkyle.

6. Emulsion selon l'une quelconque des revendications précédentes, dans laquelle le perfluoropolyéther est choisi parmi ceux qui contiennent les unités perfluorooxyalkylène suivantes, soit individuellement soit en combinaison entre elles :
(I) (CF₂-CF₂O) et (CF₂O), lesdites unités étant distribuées de façon statistique le long de la chaîne perfluoropolyéther ;
II) 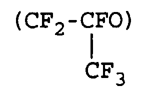 et (CFXO), où X représente F ou CF₃, lesdites unités étant distribuées de façon statistique le long de la chaîne perfluoropolyéther ;
(III) (CF₂-CF₂O) , 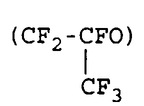 et (CFXO), où X représente F ou CF₃, lesdites unités étant distribuées de façon statistique le long de la chaîne perfluoropolyéther ;
(IV) 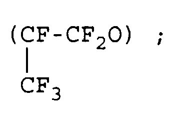
(V) (CF₂-CF₂-CF₂O) ;
(VI) (CF₂-CF₂O) ;
(VII) 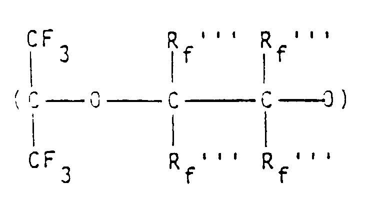 où les groupes R_{f}''', identiques ou différents les uns des autres, représentent des atomes de fluor ou des groupes perfluoroalkyle ;
(VIII) (CF₂O-CF₂-CF₂O).

7. Emulsion selon l'une quelconque des revendications précédentes, dans laquelle le perfluoropolyéther est choisi parmi ceux contenant des noyaux perfluorooxétanne de formule : 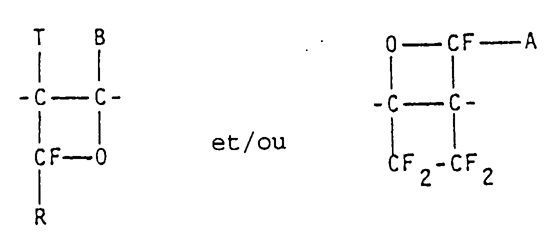 dans laquelle T, B et R, identiques ou différents les uns des autres, et A représentent des radicaux perfluorooxyalkyle, perfluoropolyoxyalkyle ou perfluoroalkyle.

8. Emulsion selon l'une quelconque des revendications 1 à 6, dans laquelle le perfluoropolyéther est choisi parmi ceux appartenant aux classes suivantes : 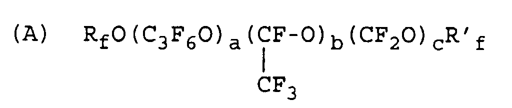
où :
- R_{f} et R_{f}', identiques ou différents l'un de l'autre, sont choisis parmi CF₃, C₂F₅ et C₃F₇ ;
- les unités entre parenthèses sont distribuées de façon statistique le long de la chaîne perfluoropolyéther ;
- a est un entier ; et
- b et c sont des entiers ou zéro ;
à la condition que, lorsque la somme (b+c) est différente de zéro, le rapport $\frac{\text{a}}{\text{b+c}}$ se situe dans une plage allant de 5 à 40 ;
(B) CF₃O(C₂F₄O)_{d}(CF₂O)ₑCF₃
où :
- les unités entre parenthèses sont distribuées de façon statistique le long de la chaîne ;
- d et e sont des entiers et le rapport d/e se situe dans une plage allant de 0,3 à 5 ;
(C) CF₃O(C₃F₆O)_{f}(C₂F₄O)_{g}(CFXO)ₕCF₃
où :
- les unités entre parenthèses sont distribuées de façon statistique le long de la chaîne ;
- X représente F ou CF₃ ;
- f, g et h sont des entiers ;
- le rapport $\frac{\text{f}}{\text{g+h}}$ se situe dans une plage allant de 1 à 50 ; et
- le rapport $\frac{\text{g}}{\text{h}}$ se situe dans une plage allant de 1 à 10.
(D) R³_{f}O(CF₂CF₂CF₂O)ⱼR⁴_{f}
où :
- R³_{f} et R⁴_{f}, identiques ou différents l'un de l'autre, représentent CF₃ ou C₂F₅ ; et
- j est un entier.

9. Emulsion selon l'une quelconque des revendications précédentes, dans laquelle le perfluoropolyéther a une masse moléculaire moyenne en nombre de 500 à 20 000, en particulier de 1000 à 10 000.

10. Emulsion selon l'une quelconque des revendications 2 et 4 à 9, dans laquelle ledit composé polyhydroxylé est choisi parmi le xylitol, le mannitol, le sorbitol, le glucose, le maltitol, le saccharose, le fructose, le diglycérol, le triglycérol et le tétraglycérol.

11. Emulsion selon l'une quelconque des revendications précédentes, dans laquelle l'agent tensioactif est choisi parmi les agents tensio-actifs cationiques, anioniques, amphotères et non-ioniques.

12. Compositions destinées à la cosmétologie et à la dermatologie, contenant une émulsion telle que définie à l'une quelconque des revendications précédentes.
